# EUROPEAN PATENT APPLICATION

(11) **EP 1 813 292 A1**
(43) Date of publication of application: **01.08.2007**
(21) Application number: 06100802.5
(22) Date of filing: 25.01.2006
(51) Int. Cl.: A61L 27/14, A61L 27/18, A61L 27/50, A61L 31/04, A61L 31/06, A61L 31/14, C08G 65/40

(54) **Surgical implant and manufacturing method**

(71) Applicant: Inion Oy, 33520 Tampere (FI)
(72) Inventor: Pohjonen, Timo, 33710, Tampere (FI); Lindgren, Totti, 28430, Pori (FI); Kaikkonen, Auvo, CM23 5PX Herts (GB); Happonen, Harri, Tampere 33820 (FI); Puhakka, Toni, Tampere 33580 (FI)
(74) Representative: Kaukonen, Juha Veikko

(57) **Abstract**

A surgical implant and a method for manufacturing the same. The surgical implant has a body comprising polyaryletherketone (PAEK). Said polyaryletherketone is a homopolymer and a first section (3) of the body comprises a polyaryletherketone that has a crystallization peak in its DSC-curve.

## Description

### FIELD OF THE INVENTION

The invention relates to a surgical implant having a body comprising polyaryletherketone (PAEK).

Further, the invention relates to a method for manufacturing a surgical implant, the implant having a body comprising polyaryletherketone (PAEK).

### BACKGROUND OF THE INVENTION

Various artificial implants that can be implanted in the organ system are known. Herein, the concept 'implant' refers to shaped pieces to be implanted in the organ system, such as membranes, fixation plates, other three-dimensional spatial pieces, fixing means, such as screws, pins, rivets, tacks, and the like, that are used to support or attach tissue or to separate tissue from other tissue while healing.

The implants can be made of biodegradable or bio-stable materials. Bio-stable implants are usually made of metal, but some bio-stable implants are made of highly crystalline polymers, such as PEEK (poly-ether-ether-ketone). The mechanical strength of implants made of bio-stable polymers is usually increased not only by a very high crystallinity rate but also by the use of reinforcing fibres, such as glass fibres and carbon fibres. These kinds of implants have a very stiff structure and, thus, they are suitable for operations where the bone tissue to be supported is exposed to high stresses. One of the most successful bio-stable highly crystalline polymers for surgical implants is PEEK-OPTIMA^{®} biomaterial launched by Victrex^{®} in 1998.

PEEK (polyetheretherketone) is probably the best-known member of the PAEK (polyaryletherketone) plastics family. PAEK plastics are semicrystalline thermoplastics that have many good properties, especially outperforming mechanical properties compared with biodegradable polymers. Additionally, PAEK materials have good thermal, chemical and radiation resistance, and good fatigue durability. Other well-known PAEK plastics are, among others, PEK (poly-ether-ketone), PEKK (poly-ether-ketone-ketone), PEEKK (poly-ether-ether-ketone-ketone) and PEKEKK (poly-ether-ketone-ether-ketone-ketone). PEEK is a polyaromatic semicrystalline thermoplastic, the typical crystallinity degree of which is 30 to 35%, the melting temperature about 343 °C, the crystallisation peak about 160 °C, and the glass transition temperature about 145 °C.

Significant advantages of PAEK plastics over metals are, for instance, the elimination of imaging artefacts, the ability to inspect tissue growth and repair using x-rays - which can often be obscured with metal parts, and, more generally in this and other applications, the avoidance of allergic tissue reaction to metallic ions. In diagnostics, as well as in postoperative evaluation, it is increasingly important to monitor the healing process by modern imaging technologies, like X-ray, CT (Computed Tomography) imaging, or MRI (Magnetic Resonance Imaging). In an X-ray image, the intensive shadow produced by a metal implant overlaps the very area important to the surgeon, making it difficult, or sometimes even impossible, to adequately gain information for judging the state of the tissue. This is similar in CT imaging where metal implants create artefacts. Some PAEK plastics are transparent to X-rays and there are no artefacts created in CT images. Because plastics are non-magnetic, MRI technologies still can be used with patients that have received a plastic implant.

An advantage is also that surface modification technologies can be more easily applied to organic surfaces like PEEK polymer than to metal surfaces. This is especially important with implants that are in direct contact with tissue or blood. A further advantage is that plastic processing technology and suitable joining technologies give more freedom in designing and shaping of surgical implants.

One of the problems associated with bio-stable implants made of highly crystalline polymers, such as polyaryletherketones, is that they are difficult to shape *in situ* into the required shape. For example, a support plate supporting fractured bone should be formed in the anatomical contour of the bone in order to give appropriate support for the bone.

### BRIEF DESCRIPTION OF THE INVENTION

An object of the present invention is to provide an implant and a method for making an implant so as to alleviate the above disadvantages.

The surgical implant of the invention is characterized in that said polyaryletherketone is a homopolymer and that a first section of the body comprises the polyaryletherketone having a crystallization peak in its DSC-curve.

Further, the method of the invention is characterized by selecting manufacturing material(s) of said implant, said material(s) comprising polyaryletherketone (PAEK), heating the PAEK in a molten state, forming the implant from said manufacturing material(s), and processing the implant so that the body has a first section that comprises polyaryletherketone having a crystallization peak in its DSC-curve.

An essential idea of the invention is to manufacture the implant at least partly from PEAK having a low degree of crystallinity.

An advantage of the implant and method of the invention is that amorphous PAEK is suited to the manufacture of a firm implant into which a permanent deformation can be bent *in situ.*

Further, the idea of an embodiment of the invention is that PAEK has an inhomogeneous form such that a second section of the body comprises the same polyaryletherketone as the first section but has a crystallization peak in its DSC-curve. An advantage is that the implant is stronger than an implant of the same design but comprising only amorphous PAEK, and has a higher elongation at break than an implant comprising only crystalline PAEK.

Further, the idea of an embodiment of the invention is that in the method the cooling rate of the body is controlled in such a manner that the PAEK present in section(s) of the body having smaller cross-sectional areas cools in an amorphous state, and the PAEK present in section(s) of the body having larger cross-sectional areas cools in a crystalline state, or that the implant is formed in a mould that has two or more sections and wherein at least one section is at a lower temperature than another section. An advantage is that the method can be carried out with normal processing steps of injection moulding, extrusion or compression moulding without any additional processing steps.

### BRIEF DESCRIPTION OF THE DRAWINGS

In the following, the invention will be described in greater detail by means of preferred embodiments and with reference to the accompanying drawings, in which
Figure 1 shows a DSC-curve of an amorphous PEEK sample,
Figure 2 shows a DSC-curve of a crystalline PEEK sample,
Figure 3 is a schematic top view of a surgical implant according to the invention,
Figure 4 is a schematic graph of tensile force versus elongation of the implant shown in Figure 3,
Figure 5 is a schematic side view of a second surgical implant according to the invention,
Figure 6 is a schematic view of a third surgical implant according to the invention,
Figure 7 is a schematic view of a fourth surgical implant according to the invention,
Figure 8 is a schematic top view of a part of a fifth surgical implant according to the invention,
Figure 9 is a schematic top view of a sixth surgical implant according to the invention,
Figure 10 is a schematic graph of tensile strength versus time of a plate made of amorphous PEEK,
Figure 11 is a schematic graph of tensile strength versus time of a plate made of crystalline PEEK, and
Figure 12 is a schematic view of the implant shown in Figure 3.

For the sake of clarity, the figures show the invention in a simplified manner. Like reference numerals identify like elements.

### DETAILED DESCRIPTION OF CERTAIN PREFERRED EMBODIMENTS OF THE INVENTION

It has surprisingly been found that it is possible to process PAEK into amorphous or nearly amorphous products that exhibit ductile behaviour instead of the rigid behaviour that has been associated with PAEK.

The process for producing amorphous or nearly amorphous products can include the following steps:
1. The temperature of PAEK is raised so high that the material reaches a molten state where it can be formed into the form of a product. This can be done, for instance, in an injection-moulding machine, an extruder, compression-moulding machine, or some other plastic processing machine known per se. Filler materials, if any, can be added to the material in case they are not added to the PAEK prior to its feeding into the processing machine.
2. At least a part of the product is cooled rapidly or quenched, whereupon the PAEK present in the part hardens into the form of a product in an amorphous state. The product may be a finished implant or a semi-finished product that is to be used in manufacturing an implant.
3. Optionally, if PAEK needs to be crystallized in some parts of the product, these parts are heat treated in order to crystallize the amorphous PAEK present in said parts.

The term 'amorphous' means that the polymer has a glass transition which is followed by an exothermic crystallization peak which is further followed by endothermic melting peak in its DSC-curve. Differential scanning calorimetry (DSC) is a standard method of characterizing the thermal transitions and the degree of crystallinity of polymers. Various aspects of amorphous PEEK has been discussed, for example, in an article 'Enthalpic relaxation in semi-crystalline PEEK', J.R. Atkinson, J.N. Hay, M.J. Jenkins, Polymer 43 (2002) 731-735.

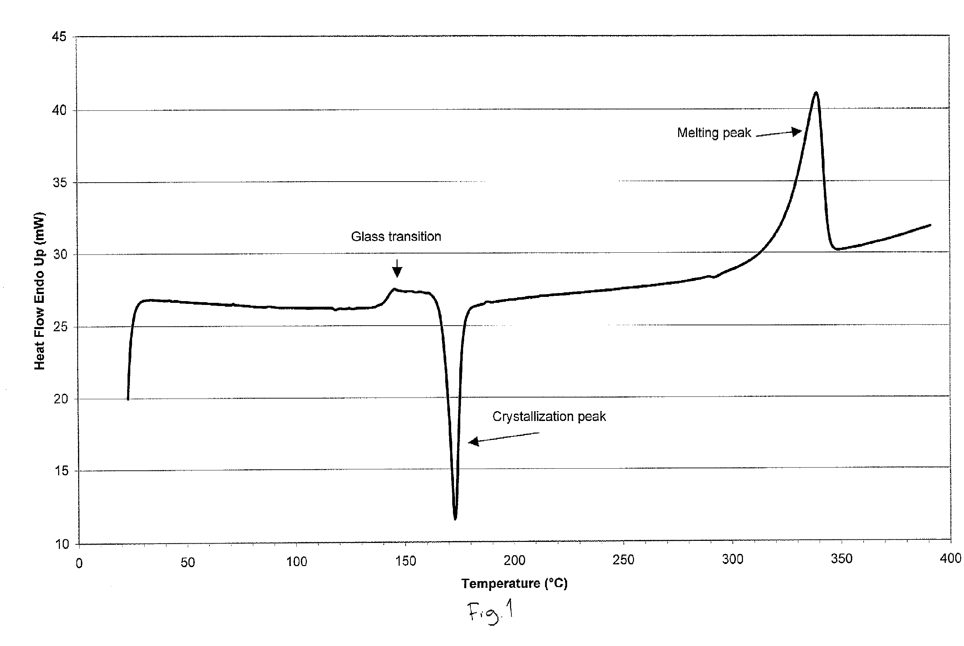

Figure 1 shows the DSC-curve of an amorphous PEEK sample. The DSC-curve shows a distinct glass transition (Tg) at around 142 to 145 °C, which is followed by a marked exothermic peak at around 160 to 170°C, which corresponds to the crystallization process that occurs upon heating in the calorimeter, and is followed by an endothermic melting peak at around 335 to 345 °C.

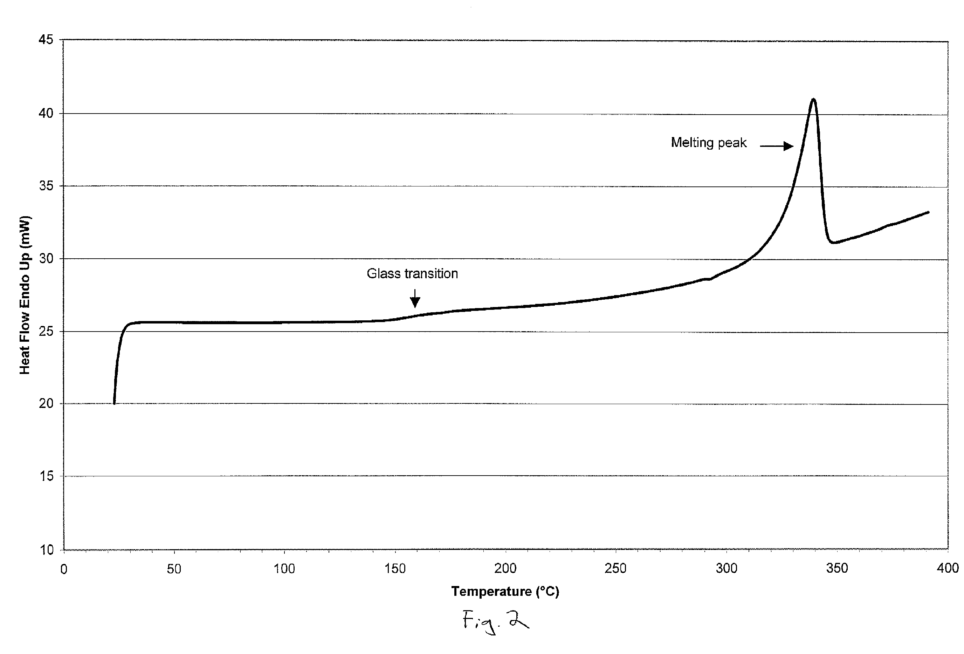

Figure 2 shows the DSC-curve of a crystalline PEEK sample. For crystalline PEEK, a small glass transition can still observed in the DSC trace but the temperature at which the process occurs is increased to about 148 to 155 °C, which is followed by only an endothermic melting peak at around 335 to 345 °C. In other words, the material has no exothermic crystallization peak before its endothermic melting peak in its DSC curve. The absence of any crystallisation exotherm indicates that crystalline PEEK is not able to crystallize further upon heating in a calorimeter. The crystallinity degree of PEEK can be estimated from the DSC-curve by integrating the areas under the crystallization exotherm and the melting endotherm, and by deducting the crystallization enthalpy from the melting enthalpy. The product is divided by the theoretical value of 130 J/g for the melting enthalpy of 100% crystalline PEEK. The crystallization enthalpy of substantially amorphous PEEK-OPTIMA^{®} LT polymers is typically from about 20 J/g to about 30 J/g at a DSC heating rate of 20°C/min.

Amorphous PAEK is usually optically amorphous, i.e. it has a translucent appearance. Therefore, PAEK can be recognized as amorphous on the grounds of its colour and light transmittance. Amorphous PAEK is transparent, whereas crystalline PAEK is opaque. Especially amorphous PEEK-OPTIMA^{®} LT polymers are transparent and dark brown, whereas crystalline PEEK-OPTIMA^{®} LT polymers are opaque and pale brown. When an amorphous PEEK-OPTIMA^{®} LT sheet, the thickness of which is about 0.2 to 2.0 mm, is placed on white paper with black print on it, the print can easily be seen through the sheet. Contrary to this, the print cannot be seen through a crystalline PEEK-OPTIMA^{®} LT sheet of the same thickness.

Other scientific techniques available for measuring the degree of crystallinity of PAEK polymers include density measurements, Fourier Transformed Infra-red Spectroscopy (FTIR), Wide Angle X-ray Scattering (WAXS).

The crystallinity degree of amorphous PEAK is preferably less than 18%, more preferably less than 15%, even more preferably not more than 10%, and most preferably not more than 5%. The lower the crystallinity degree the better the shaping characteristics of the PAEK. The crystallinity degree is naturally adjusted according to the requirements set on the implant.

The term 'crystalline' means that the polymer has no exothermic crystallization peak in its DSC-curve. The degree of crystallinity of crystalline PAEK is preferably at least about 20%, more preferably 25% or more.

It is to be noted that with the addition of a filler material by a compounding process, which may be in the form of carbon or glass fibres, or Kevlar fibres, or whisker fibres, for instance, or any other fibres made of polymers, metals and/or ceramics, the strength of natural unfilled PAEK may be increased significantly in order to cater for greater stress-demanding applications. Stiffness tailoring to bone is also possible with the judicious selection of fibres or other fillers at an appropriate concentration. Such bone modulus matching may be important in applications for which stress shielding should be minimized. Amorphous or crystalline sections separately, as well as both of them, can be filled and reinforced.

### Example 1

Regular sized dog-bone shaped tensile test specimens were produced using conventional injection moulding techniques. The length of the specimens were 100 mm, thickness 1.2 mm and width 10.5 mm. Pellets of PEEK-OPTIMA^{®} LT1 and PEEK-OPTIMA^{®} LT3 polymers were obtained from Invibio Ltd, of Thornton Cleveleys, England. Prior to injection moulding, the raw materials were dried at 120 °C for 10 hours to remove residual moisture.

The test specimens were moulded with a generally known Kraus-Maffei KM50C2 electro-hydraulic injection moulding machine. Barrel temperatures were between 355 and 375 °C for both PEEK-OPTIMA^{®} LT1 and PEEK-OPTIMA^{®} LT3 materials.

Mould temperature was 55 °C for PEEK-OPTIMA^{®} LT1 material and 40 °C for the more easily flowing PEEK-OPTIMA^{®} LT3 material. In other words, the mould temperature was substantially lower than the glass transition temperature T_{g} of the material to be processed. This resulted in rapid cooling and solidification of the material in the mould. Because of the rapid cooling, the material lacked time to generate a sufficient number of crystalline cores to be properly crystallized, and the material solidified into an amorphous state.

Accordingly, cooling of the material was implemented with a very cold mould. Similarly, a very fast cool down of the polymer melt after extrusion nozzle can be used in extrusion. An alternative is to use extremely cold liquids or gases for cooling the mould or the polymer melt, for example liquid carbon dioxide or nitrogen, which can be applied directly to the surface of a component or a semi-finished product or to a tool or a mould. The semi-finished product or the component may also be immersed in these liquids, gases or solid states thereof, such as dry ice, for example.

Some of the amorphous test specimens were crystallized post moulding by heat treatment in order to obtain reference test specimens. Since PAEK crystallizes rapidly, crystalline mouldings can be also obtained if the temperature of the mould cavity is heated to the range of crystallization peak temperature during the injection moulding process. The post moulding crystallization heat treatments of amorphous specimens were carried out in a vacuum furnace according to the following program: first, the temperature was raised to the value 150 °C, where it was kept for 16 hours. The temperature was then raised to the value 175 °C for four hours, after which the temperature was raised to the value 200 °C, where it was kept for 16 h. Finally, the temperature was allowed to fall freely to room temperature.

The test specimens were subjected to tensile stress tests in accordance with the ISO 527 Plastics tensile stress test standard 'Determination of tensile properties', in order to enable the study of the differences between amorphous PEEK and heat-treated crystalline PEEK. The results are presented in Tables 1 and 2 below.

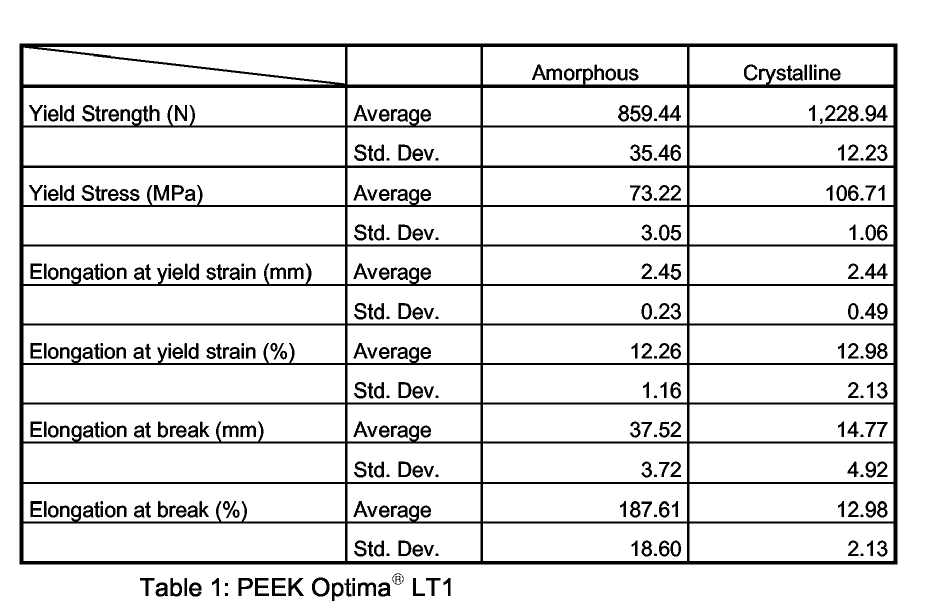

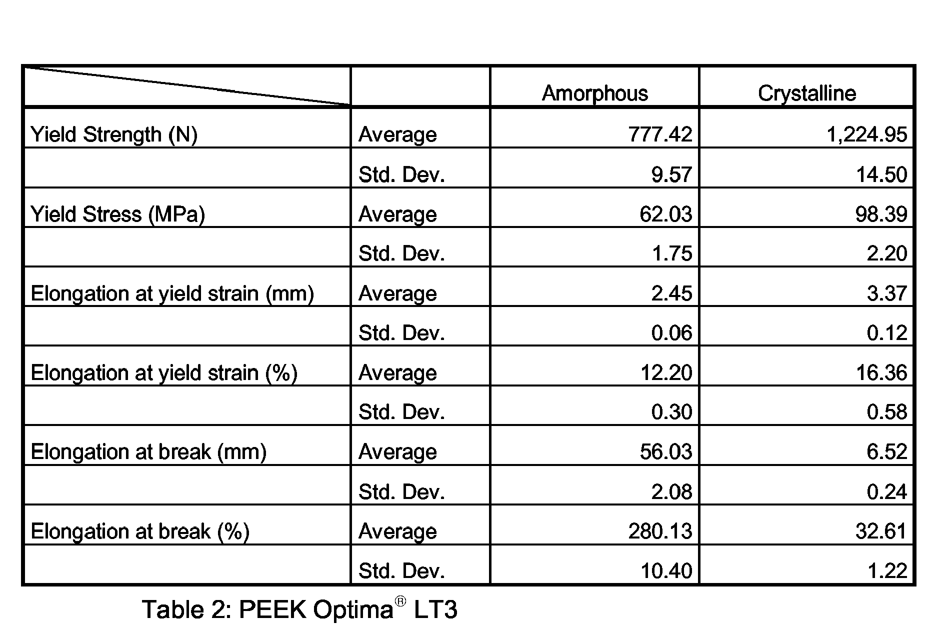

The degrees of crystallinity of the test specimens were determined with a TA Instruments Q1000 DSC device at a heating speed of 20°C/min. A theoretical value of 130 J/g was employed for the melting enthalpy of a 100% crystallized PEEK polymer. The calibration of DSC was carried out with a pure Indium standard sample, whereby the measured melting temperature of Indiumin was 157.35°C and the melting enthalpy 28.39 J/g.

The degree of crystallinity of amorphous PEEK-OPTIMA^{®} LT1 test specimens was 14.8%, whereas the degree of crystallinity of amorphous PEEK-OPTIMA^{®} LT3 test specimens was 9.7%. The degree of crystallinity of crystallized PEEK-OPTIMA^{®} LT1 test specimens was 34% and the degree of crystallinity of crystallized PEEK-OPTIMA^{®} LT3 test specimens was 33%. Amorphous PEEK was optically amorphous, in other words, it was manifestly translucent. In contrast, crystallized PEEK was opaque.

On the basis of the results, crystalline PEEK is distinctly harder than amorphous PEEK. The elastic deformation range for both phases is equal in size. On the basis of the elongation at break of the tensile strength test, amorphous PEEK is considerably more ductile than PEEK. Amorphous PEEK is better suited than the crystalline one to an application wherein a product has to be given a permanent deformation without, however, weakening the strength grades. Such a product may be for instance a plate-like implant, whose shape has to be bent suitable for the site of attachment in some operations, for the shape of the surface of a bone, for example.

The reduction in the mould temperature to the level of room temperature, to about 20 to 25 °C, may result in degrees of crystallinity below 5%.

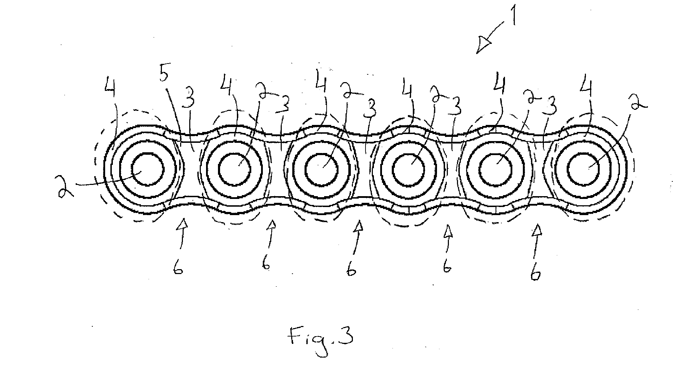

Figure 3 is a schematic top view of a surgical implant according to the invention. The implant is a fixation plate 1. The fixation plate 1 is usually employed in such a manner that it is fixed to both sides of the fractured or splintered point of the bony tissue by using known fixing elements, such as screws or pins, fitted through fixing holes 2. The fixation plate 1 keeps the bone in the correct position allowing it to heal in the best possible manner.

The fixation plate 1 shown in Figure 3 comprises a total of six fixing holes 2 arranged in a row at equal distances and protruding through the body 5 of the plate 1. Forming sections 6 are arranged between the holes 2. Usually, fixation plates 1 are made in a variety of sizes and lengths with a variable number of screw holes. A fixation plate can also be shaped like the letter L, T, X or Y, or designed in some other shape.

The plate 1 is made of material comprising a PEEK homopolymer, i.e. a polymer that is formed from only one type of monomer. PEEK can be readily melt-processed by injection moulding, extrusion and compression moulding, for example.

The plate 1 shown in Figure 3 consists of eleven sections arranged consecutively in the longitudinal direction of the plate 1. First sections 3 comprise mainly amorphous PEEK and second sections 4 comprise mainly crystalline PEEK. For sake of clarity, the second sections 4 are surrounded by broken lines in Figure 3.

The plate 1 has the advantage that during shaping of the plate 1 it deforms only or at least mainly at the forming sections, i.e. between the holes, while the holes 2 do not deform practically at all, as shown in Figure 12. Furthermore, the plate design comprising both crystalline and amorphous sections is stronger than a plate comprising only amorphous PEEK, and has a higher elongation at break than a plate comprising only crystalline PEEK.

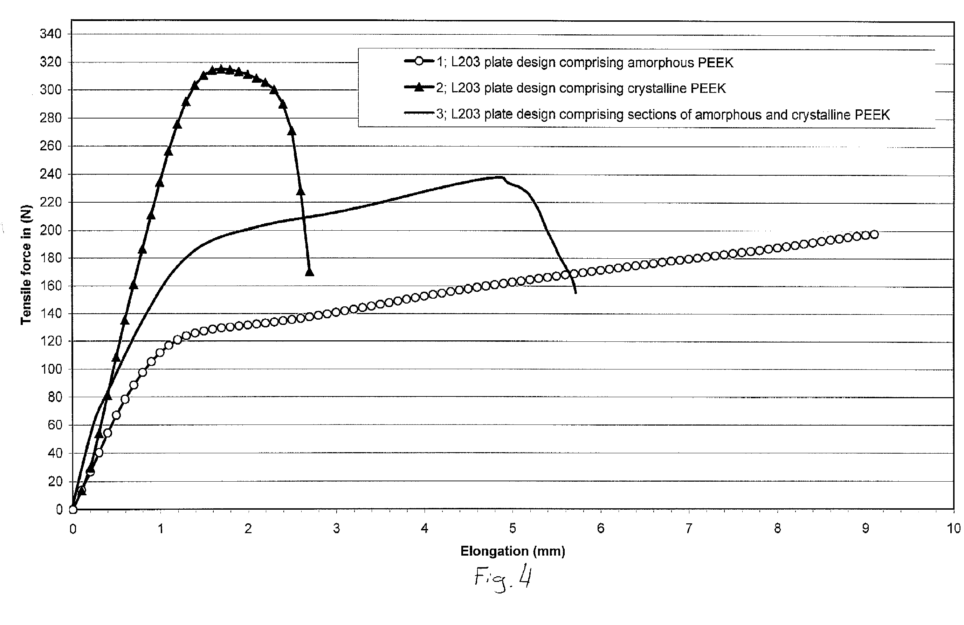

Figure 4 is a schematic graph of tensile force versus elongation of the plate 1. The degree of crystallinity of the amorphous sections was 9.5% and the degree of crystallinity of the crystalline sections was 33.6%. As a reference, plates with the same design but consisting of wholly amorphous PEEK were manufactured and tested. Also plates with the same design but consisting of wholly crystalline PEEK were manufactured and tested. Figure 4 shows clearly that the plate design comprising both crystalline and amorphous sections is stronger than a plate comprising only amorphous PEEK, and has a higher elongation at break than a plate comprising only crystalline PEEK.

The processing parameters of the plate 1 can be controlled in such a way that the first sections 3 are amorphous with excellent ductility and excellent shaping characteristics, whereas the second sections 4 in the direct vicinity of the holes 2 are crystalline. The ductile characteristic of the amorphous sections 3 allows the plate used in osteosynthesis to be bent and contoured to the bone shape. The plate 1 is thus easy to shape and yet supports the healing bone sufficiently.

In an embodiment of the invention, the crystallinity degree of PAEK in the first section of the body is at least 15% lower than the crystallinity degree of PAEK in the second section of the body. This way the mechanical properties of the first section differ clearly from those of the second section, so that when the implant is bent, the bending takes place only in the first section whereas the second section does not bend.

In the embodiment shown in Figure 3, the whole plate 1 is made of PEEK. The plate 1, or some other implant, can also made from some other PAEK, for example PEK, PEKK, PEEKK, and PEKEKK. It is also possible that some parts of an implant according to the invention are made from some other material than PAEK. Furthermore, in an embodiment of the invention, not all of the holes are surrounded with crystalline PAEK but some of them are made from an amorphous material. This makes the plate even easier to be formed in anatomical contours. In another embodiment, some of the sections between the holes 2 are made of crystalline PAEK. This increases the stiffness of the plate between said holes.

A method for manufacturing the plate 1 is described in the following example 2.

### Example 2

The plate 1 according to Figure 3 was made from the material PEEK-OPTIMA^{®} LT3 by first injection moulding the entire amorphous semi-finished product in the shape of plate 1 by using the manufacturing parameters and injection moulding devices presented in Example 1. Thus, the temperature of the mould was so low that PEEK hardened in an amorphous state.

Next, the amorphous semi-finished product, removed from the mould, was adapted into a heat treatment device comprising heating heads to be heated to a high temperature. Each heating head comprised a contact surface shaped in the shape of the part of the semi-finished product to be heated with said heating head. The heating heads were manufactured from an electrically conductive metal, the heating itself being implemented with electric resistances. The heating heads were pushed against the sites of the semi-finished product to be crystallized and hold in this position approximately 10 to 30 sec. In this case, the sites to be crystallized were located in the vicinity of each fixing hole 2. The areas of the semi-finished product to be left amorphous were not in contact with the heating heads; instead, they were in free contact with the surrounding air. The heating heads raised the temperature of the areas to be crystallized in such a manner that the PEEK comprised by them could be organized into a crystalline state. The temperature of the heating heads is typically at least 230 °C and is chiefly determined on the basis of the thickness, shape and manufacturing material of the semi-finished product. This being so, the entirely amorphous semi-finished product formed into a plate 1, which was non-homogenous comprising amorphous parts 3 and crystalline parts 4. At this stage of the manufacture of the plate 1, external cooling can be utilized, by means of which the parts of the plate to be kept amorphous are cooled while the parts to be crystallized are heated. Cooling can be implemented for instance by dousing said parts of the semi-finished product with a suitable fluid or gas or by using cooling elements, generally made from metal, which are pressed against the parts of the semi-finished product to be cooled. The semi-finished product and the heating heads fastened thereto can also be entirely immersed in a cooling liquid. The time required for crystallizing the parts of the semi-finished product depends not only on the temperature employed but also on the thickness, shape and manufacturing material of the semi-finished product.

The plates 1 according to Figure 3 can be fastened to a bone for instance with screws manufactured from crystallized PEEK or another PAEK. The screws can also be injection moulded into a cold mould in accordance with the principle presented in Example 1, whereby they remain substantially amorphous. An advantage of a substantially amorphous PAEK screw is its ductility, owing to which the screw can be tacked into the bone with an instrument suitable for the purpose, such as Inion^{®} Tacker^{™}, without any fear of the screw breaking when the hammer of the instrument hits the screw head.

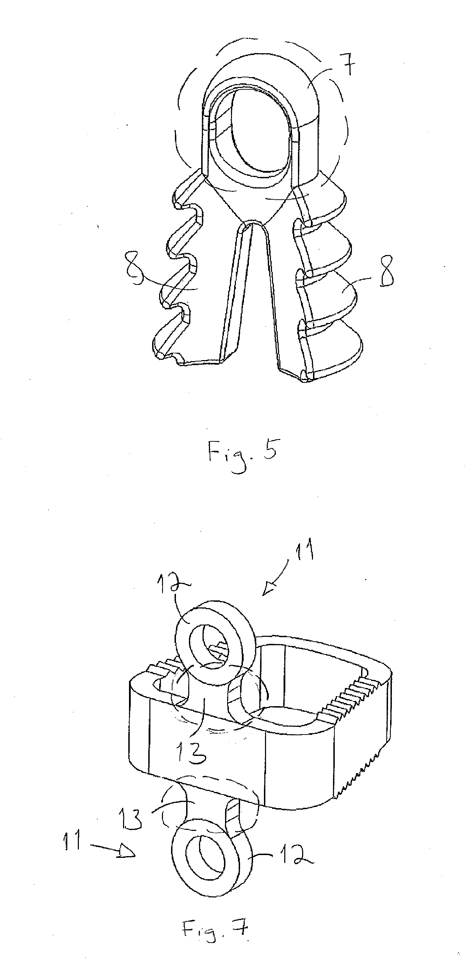

The elasticity of amorphous PAEK and the strength of crystalline PAEK can also be utilized in anchors employed for fastening of ligaments and tendons to bone, such as ACL anchors (anterior cruciate ligament), PCL anchors (posterior cruciate ligament), or other soft tissue to bone fixation devices, such as tacks for sutureless repair of tears of labrum or rotator cuff of the shoulder, suture anchors for soft tissue to bone attachment, and the like. Figure 5 shows an anchor of this type. The annular part 7 of the anchor, which during installation is subjected to tensile press stresses, is made from amorphous PEEK, whereas the toothed legs 8 that bite into the bone are made from crystalline PEEK. In this manner, the product is provided with an extremely good functionality during installation and an extremely durable fastening to the bone. For sake of clarity, the section of amorphous material is surrounded by broken lines in Figure 5.

An anchor was made in accordance with the principle presented in Example 2. The degree of crystallinity of the amorphous area, measured from the anchor with DSC, was 15%, the degree of the crystalline area being 32%.

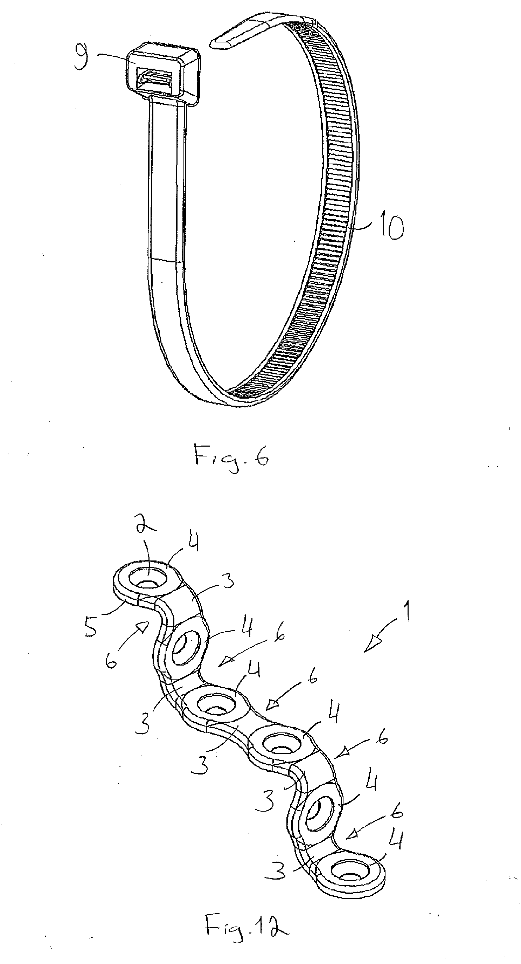

Figure 6 shows a third implant according to the invention. The implant is a so-called cable tie. It comprises an amorphous part and a crystalline part. The implant can be used for instance for rapid and secure fastening of a sternum cut after open chest heart surgery. A cable tie is also a fast and secure fastening means for fixing of longitudinal fissures in bones. Cable ties are also useful for the reduction of comminuted bones. If necessary, cable ties can be used together with conventional rigid bone fracture fixation means, such as metal plates and screws. Furthermore, small cable ties are useful for litigation of blood vessels, i.e. tying a duct of blood vessel with a ligature as to prevent bleeding during surgery.

The lock 9 of the cable tie is of a crystalline PAEK material, since the lock 9 is the very part that is subject to the strongest stresses. The band 10 is made from an amorphous PAEK material in order to be easily bendable into shape and in order to endure tensile load, which may even exceed the yield limit of the material to some degree. In this case, the band 10 slightly stretches, but does not break.

### Example 3

The cable tie according to Figure 6, or any other implant having a shape comprising parts/sections having substantially different cooling rates, for example a fixation plate having thick sections of material around the fixing holes, can be manufactured for instance by injection moulding in such a manner that PAEK is injected into an isothermal mould. Since the ratio of the outer area of the lock 9 to its volume is substantially larger than the corresponding ratio in the band 10, the material constituting the lock 9 cools substantially slower than the material constituting the band 10. For this reason, the material constituting the lock 9 has more time to orientate into a crystalline state, as a result of which the lock 9 of the cable tie, cooled to room temperature, is composed of crystalline PAEK. In contrast, the band 10 cools very rapidly, owing to which the band 10 of the cable tie, cooled to room temperature, is composed of amorphous PAEK.

The cable tie according to Figure 6, or any other elongated or plate-like implant, can also be manufactured by moulding the implant or the semi-finished product employed in the manufacture thereof in a mould, one part of the mould surface of whose mould cavity is at a lower temperature than a second part of the mould surface of said mould cavity. The selection of suitable mould temperatures makes the PAEK, pressed against the colder mould surface, cool so rapidly that it remains in an amorphous state. In contrast, the PAEK, pressed against the hotter mould surface, cools slower and has time to crystallize.

The mechanical differences between amorphous and crystalline PAEK can also be utilized in spinal cages. The spinal cage shown in Figure 7 comprises two projections 11 having holes. The screws or the like for fastening the spinal cage to tissue are adapted through rings 12 in the projections to the tissue. The rings 12 are arranged at the ends of arms 13. The arms 13 are of amorphous PAEK, owing to which they can be easily bent into an angle required by the fastening. This allows the surgeon to use the spinal cage more freely in different operational situations. In contrast, the other parts of the spinal cage can be of crystalline PAEK, which is rigid and hard. For sake of clarity, the sections of amorphous material are surrounded by broken lines in Figure 7.

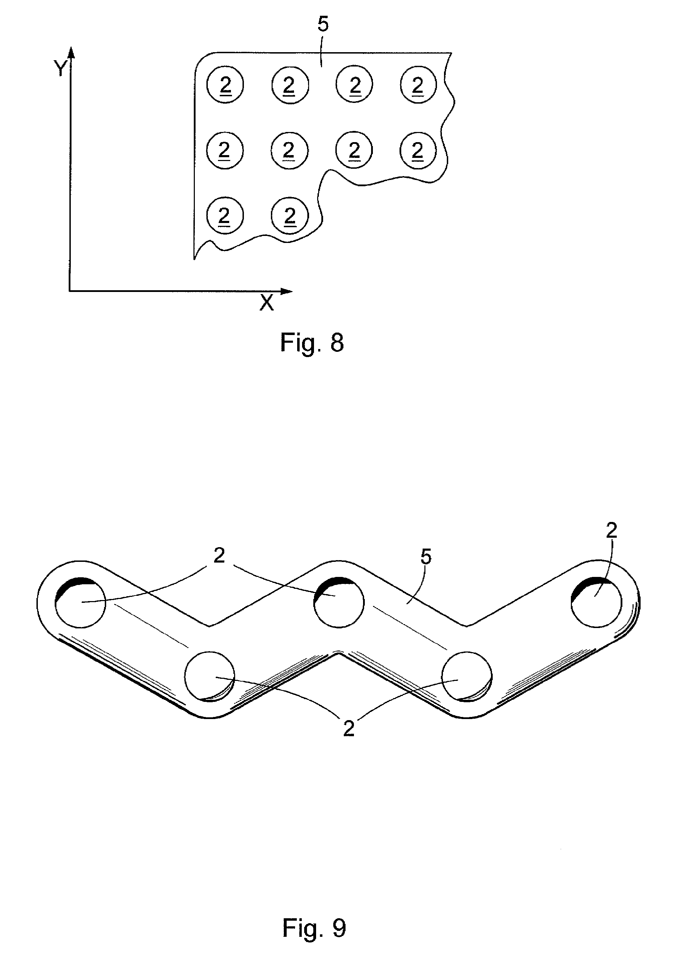

Figure 8 shows part of a mesh plate according to the invention, comprising a plurality of fixing holes 2 arranged in two main directions X, Y. The mesh plate is made from PAEK that is amorphous throughout the entire body 5 of the plate. Such a plate is easy to mould into the fastening point, which may be a cranial bone or the acetabulum.

Other implants can naturally also be made entirely from an amorphous PAEK material, such as the plate 1 shown in Figure 1, for example. Such implants can be used for instance when the implant, when fastened in place in the organism, is not subjected to large loads or if the implant has to be extremely easily and versatilely mouldable. Such applications exist in facial and cranial surgery, for example. For example, amorphous PAEK plates can be bent a plurality of times without the mechanical properties of the plate suffering; as opposed to conventional titanium plates.

In some other embodiments of mesh plates, the immediate surroundings of some or even all of the fixing holes are of crystalline PAEK, the spaces between the crystalline areas being of amorphous PAEK.

The plate according to Figure 9 can be used for the fixation of fractures of various tubular bones, the plate having a body 5 in zigzag form and comprising a plurality of fixing holes 2. The plate can be manufactured for instance by injection moulding carbon fibre reinforced PEEK into a cooled mould in such a manner that the PEEK matrix polymer remains amorphous. The plate could easily be deformed to conform to the curvature of the bone by bending the plate with pliers at room temperature. However, the screws intended for fastening the plate and made from PAEK are preferably injection moulded by injecting PAEK into a hot mould, which makes them crystalline and extremely rigid screws that bite well even into hard bone. Let it be reminded in this context that the plate can naturally also be fastened to the bone with fixing means made from another material.

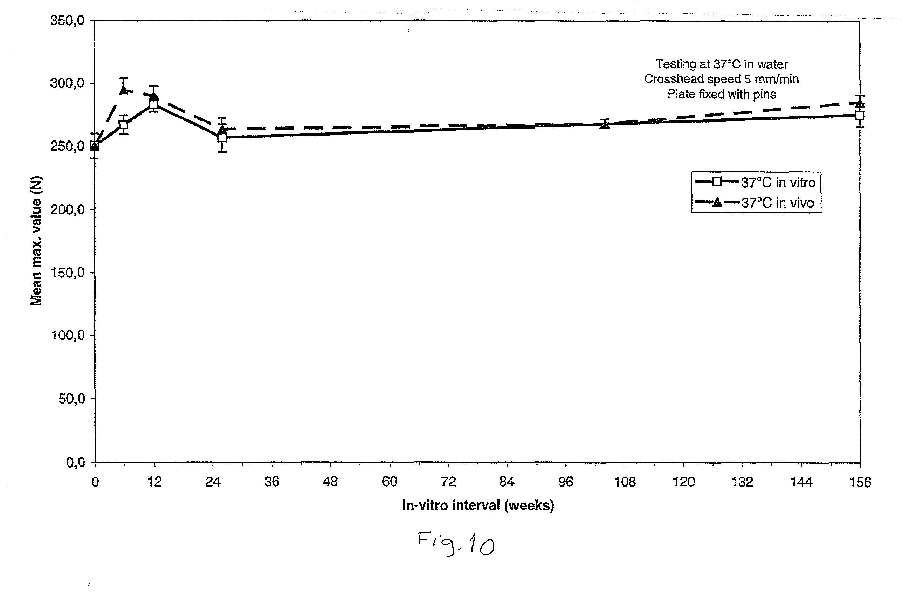

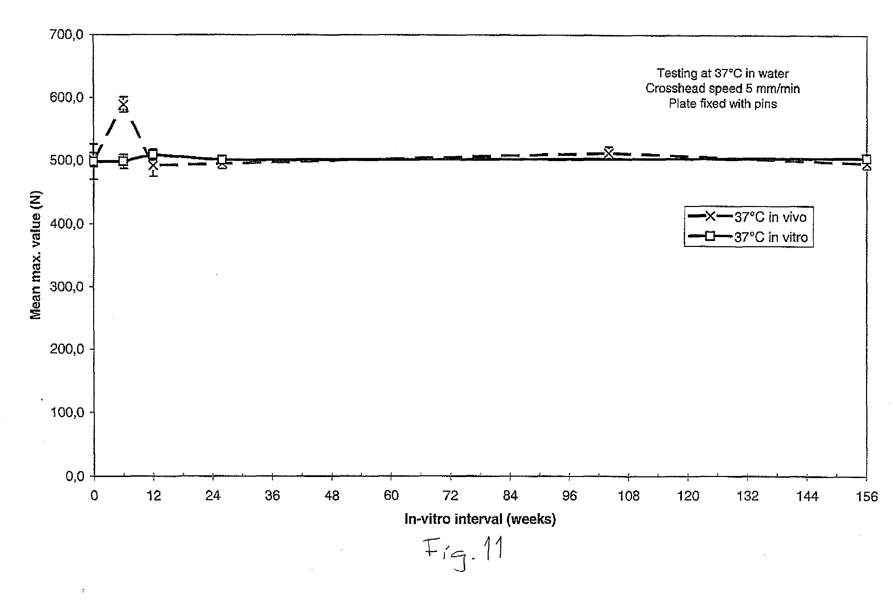

Crystalline PEEK is known to be a biocompatible, bio-stable and hydrolytically durable material. In contrast, no research on the biocompatibility and bio-stability of PEEK can be found in literature. To find out the biocompatibility and hydrolytic durability of amorphous PEEK, amorphous plates having a degree of crystallinity below 15% were injection moulded with an Inion CPS^{®} PLT-1015 plate mould from a PEEK-OPTIMA^{®} LT3 polymer. Some plates were crystallized by heat treatment in the manner described in Example 1, yielding a degree of crystallinity of more than 25%. The plates were sterilized by gamma radiation using a 25 kGy minimum dose. Some plates were implanted *in vivo* in the subcutis of sheep under the hide and some plates were subjected to an *in vitro* hydrolysis test, wherein the plates were stored in a laboratory in a physiological phosphate buffer solution in an incubator at the temperature of 37 °C for a maximum of 3 years. Figures 10 and 11 show the durability of the tensile strength of amorphous and crystalline PEEK plates *in vitro* and *in vivo.* Amorphous plates turned out to have a hydrolytic durability and biocompatibility fully equal to crystalline PEEK plates. It should be noted here that PAEK can be sterilized using all known methods, equipment and principles, e.g. autoclaves, ethylene oxide, plasma sterlization, UV-sterilization, gamma sterilization, electron sterilization, and the like. Due to the high processing temperatures PAEK product is sterile after its shaping. Therefore, if the product is moved immediately from the processing equipment to a sterile packaging, a sterile and packed product can be obtained. This way a separate sterilization step can be avoided.

The shape stability of fixation plates is an essential requirement in facial and cranial surgery, for example. The plates have to be bent often into complex forms, and the plates have to maintain their shape accurately under physiological circumstances. The stability of the angle of curvature of PEEK plates was tested as follows: amorphous (degree of crystallinity less than 15%) and crystalline (degree of crystallinity more than 30%) 1.3 mm thick, 7 mm wide and 37 mm long plates for craniomaxillofacial (CMF) surgery (shape similar to Inion CPS^{®} 2.0 mm system PLT-1038 plate) having 6 holes with a hole spacing of 6 mm and a hole diameter of 2 mm were used as components to be tested. The amorphous plates were manufactured from the PEEK^{®} Optima LT1 material by injection moulding. The crystalline plates were manufactured by crystallizing injection-moulded amorphous plates by heat treatment in the manner described in Example 1. The test arrangements were as follows:

Five parallel experiments were performed in the test in one test series. The test series to be tested were: a) amorphous and b) crystalline. The components to be tested were bent into an angle of 90° with palte bending pliers (Inion^{®} Ins-9024) in the middle of the plates between the holes.

The bending into angle was performed by first bending the plate into an angle of 'over' 115°, which was checked by measuring it by means of an angular scale drawn on paper, after which the angle was adjusted to the value 90°. This angle is also measured with the angular scale. The bent components were kept at room temperature for an hour, after which the angle was checked on a measuring scale. The components were then applied to *in vitro* circumstances in an incubator (phosphate buffer solution pH 7.4, temperature 37 °C).

The stability of the angle was followed during six weeks by performing measurements at the following intervals from the placement into *in vitro* circumstances: 24 hours, 48 hours, 96 hours, 1 week, 3 weeks, 6 weeks. The measurements were made by means of a measuring scale. Table 3 shows a summary of the stability of the angle of curvature of the plates.

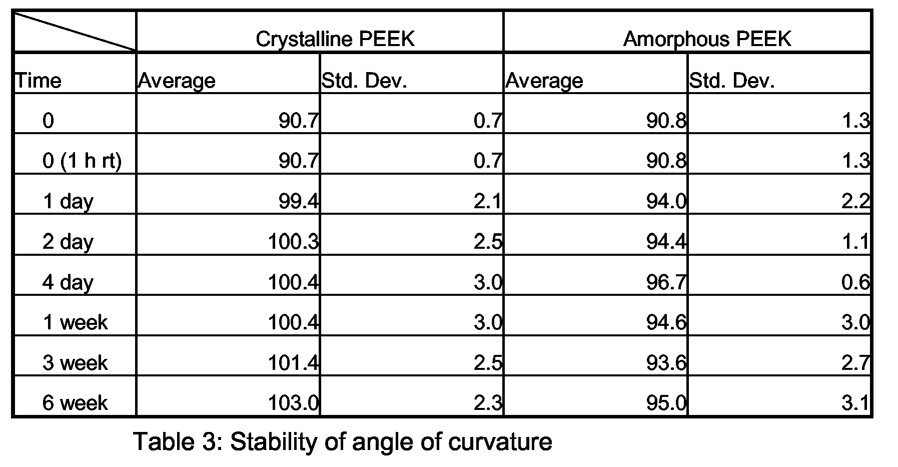

On the basis of the results, amorphous PEEK retains the shape moulded therein better than does crystalline PEEK.

It will be obvious to a person skilled in the art that as technology advances, the inventive concept can be implemented in various ways. The invention and its embodiments are not limited to the examples described above but may vary within the scope of the claims.

## Claims

1. A surgical implant having a body comprising polyaryletherketone (PAEK), **characterized in that** said polyaryletherketone is a homopolymer and that a first section (3) of the body comprises polyaryletherketone having a crystallization peak in its DSC-curve.

2. A surgical implant as claimed in claim 1, **characterized in that** the polyaryletherketone in said first section (3) is optically amorphous.

3. A surgical implant as claimed in claim 1 or 2, **characterized in that** the degree of crystallinity of the polyaryletherketone does not exceed 18%.

4. A surgical implant as claimed in any one of claims 1 to 3, **characterized in that** the whole implant is made from polyaryletherketone having a crystallization peak in its DSC-curve, optionally filled with a filler material.

5. A surgical implant as claimed in any one of claims 1 to 3, **characterized in that** the homopolymer has an inhomogeneous form in such a manner that a second section (4) of the body comprises the same polyaryletherketone as the first section (3) but has no crystallization peak in its DSC-curve.

6. A surgical implant as claimed in claim 5, **characterized in that** the polyaryletherketone having no crystallization peak in its DSC-curve has a degree of crystallinity of at least about 20%.

7. A surgical implant as claimed in claims 5 or 6, **characterized in that** the implant is a plate (1) for supporting tissues, the plate (1) comprising at least two fixing holes (2) that extend through the plate (1) from an upper surface to a lower surface of the plate (1), and a forming section between the fixing holes (2), said first section (3) being arranged in at least one forming section, and said second section (4) being arranged in the vicinity of at least one the holes (2).

8. A surgical implant as claimed in any one of claims 1 to 7, **characterized in that** the polyaryletherketone in the first section (3) has a degree of crystallinity not exceeding 10%.

9. A surgical implant as claimed in any one of claims 1 to 8, **characterized in that** the polyaryletherketone in the first section (3) has a degree of crystallinity not exceeding 5%.

10. A surgical implant as claimed in any one of claims 5 to 7, **characterized in that** the polyaryletherketone in the second section (4) has a degree of crystallinity of 25% or more.

11. A surgical implant as claimed in any one of claims 5 to 7, **characterized in that** the degree of crystallinity of the polyaryletherketone in the first section (3) is at least 15% lower than the degree of crystallinity of the polyaryletherketone in the second section (4).

12. A surgical implant as claimed in any one of claims 1 to 11, **characterized in that** it is a tendon anchor.

13. A surgical implant as claimed in any one of claims 1 to 11, **characterized in that** it is a tack used for sutureless soft tissue to bone fixation.

14. A surgical implant as claimed in any one of claims 1 to 11, **characterized in that** it is a suture anchor used for soft tissue to bone fixation.

15. A surgical implant as claimed in any one of claims 1 to 11, **characterized in that** it is a spinal cage.

16. A surgical implant as claimed in any one of claims 1 to 11, **characterized in that** it is a cable tie.

17. A surgical implant as claimed in any one of claims 1 to 11, **characterized in that** it is a mesh plate.

18. A surgical implant as claimed in any one of claims 1 to 15, **characterized in that** said polyaryletherketone is selected from the following group: polyetheretherketone (PEEK), polyetherketone (PEK), polyetherketoneketone (PEKK), polyetheretherketoneketone (PEEKK) and polyetherketoneetherketoneketone (PEKEKK).

19. A surgical implant as claimed in any one of claims 1 to 18, **characterized in that** said polyaryletherketone is filled with reinforcing fibres.

20. A Method for manufacturing a surgical implant, the implant having a body comprising polyaryletherketone (PAEK), the method comprising steps of:
selecting manufacturing material(s) of said implant, said material(s) comprising polyaryletherketone (PAEK),
heating the polyaryletherketone in a molten state,
forming the implant from said manufacturing material(s), and
processing the implant so that the body has a first section (3) that comprises polyaryletherketone having a crystallization peak in its DSC-curve.

21. A method as claimed in claim 20, **characterized in that** the polyaryletherketone in said first section (3) is optically amorphous.

22. A method as claimed in claim 20 or 21, **characterized in that** the degree of crystallinity of the polyaryletherketone does not exceed 18%.

23. A method as claimed in claim 22, **characterized by** processing the implant in such a manner that essentially all the polyaryletherketone in the body has a degree of crystallinity not exceeding 18%.

24. A method as claimed in any one of claims 20 to 23,
**characterized by**
processing the implant in such a manner that essentially all the polyaryletherketone in the body has a crystallization peak in its DSC-curve,
heating selected section(s) of the body such that an inhomogenous structure of the body is generated/established, whereby
the body comprises one or more first sections (3) where the polyaryletherketone has a crystallization peak in its DSC-curve, and one or more second sections (4) where the polyaryletherketone has no crystallization peak in its DSC-curve.

25. A method as claimed in claim 20, **characterized by** forming the body of the implant, the body having essentially distinguishable cross-sectional areas,
controlling the cooling rate of the body in such a manner that the polyaryletherketone comprised by the section(s) of the body having smaller cross-sectional areas cools in an amorphous state, and the polyaryletherketone comprised by the section(s) of the body having larger cross-sectional areas cools in a crystalline state.

26. A method as claimed in claim 25, **characterized by** forming the implant in a mould that comprises two or more sections and wherein at least one section is at a lower temperature than another section.
